# EUROPEAN PATENT APPLICATION

(11) **EP 0 951 901 A2**
(43) Date of publication of application: **27.10.1999**
(21) Application number: 99200129.7
(22) Date of filing: 19.01.1999
(51) Int. Cl.: A61K 7/20

(54) **Gel containing hydrogen peroxide**

(30) Priority: 20.01.1998 CH 12498
(71) Applicant: Intramed AG, 6301 Zugo (CH)
(72) Inventor: Weissmahr, Joseph, 8053 Zurigo (CH)
(74) Representative: Fiammenghi-Domenighetti, Delfina

(57) **Abstract**

A dental gel is described for the hygiene and disinfection of the teeth and the gums, obtained by a process of "thickening" of hydrogen peroxide using suitable gelling substances.

## Description

The present invention relates to the field of contact substances (pastes, gels, liquids) for hygiene and disinfection of the oral cavity, in particular the teeth and the gums.

It is known that, according to the current state of the art, pastes, gels and liquids are known containing various chemical substances mixed together which bring about, by contact action, both cleaning and partial disinfection of various parts of the oral cavity (especially the teeth and the gums), by carrying out an action, which is moreover very limited, of removal of the bacterial plaque; however, for genuine removal of this plaque, the only effective means remains the intervention with mechanical means by an orthodontist.

In particular, the various toothpaste pastes or gels - as are currently formulated - do not allow the active substances to be applied directly, continuously or in a suitable concentration at least partly combined with a mechanical action on plaque.

It is known that hydrogen peroxide exerts disinfectant, whitening and anti-plaque action on account of the oxygen produced thereby, which gives rise to oxidations at the membrane level.

This substance, mixed with many other substances, is also found, rarely, in toothpastes, mainly for its properties of whitening teeth.

It is also known that hydrogen peroxide is characterized by very high instability, as a result of which it also has a tendency spontaneously to decompose, especially when introduced in mixtures of various other substances which interact with each other and with it (including surfactants), such as in toothpastes.

Moreover, increasing the hydrogen peroxide concentration in order to reduce the consequences of its instability can induce problems of intolerance.

The possibility of using hydrogen peroxide as a solution in the liquid state in order to carry out cleansing, irrigations and rinsings is thus limited by many drawbacks.

Specifically, using a solution of this type involves a drastic reduction in the efficacy and duration of the oxidation reactions, even in the presence of high concentrations of the substance, on account of the almost immediate dilution of this substance, its random dispersion in the oral cavity, the objective difficulty of directing the liquid and of keeping it on the points to be treated by means of a toothbrush, in particular the neck of the teeth and any gingival pockets, and the risk of ingestion.

The inventor of the present patent has thus sought a solution which could make it possible to extend the disinfectant action of hydrogen peroxide for a longer period and which would, at the same time, allow the product to reach less accessible zones, such as the interstices between the teeth, by applying it using a brush, even one of common type, for cleaning the teeth, thereby overcoming all of the abovementioned limitations, including those of tolerability.

The inventor has thus devised and produced for this purpose a contact substance in gel form consisting of the direct "gelation" of hydrogen peroxide by means of a simple process of "thickening" organic substances which have the property of stabilizing the hydrogen peroxide over time and subsequently releasing the nascent oxygen by the action of mechanical rubbing.

Thus, the invention is one of a direct gelation of a single active substance, namely hydrogen peroxide, rather than of a mixture of various substances.

The subject of the present invention is thus a dental gel according to the preamble of the attached Claim 1, characterized by the characterizing part of that claim.

A more detailed description of a few examples of embodiments of the dental gel according to the present invention will now be given.

A composition suggested by the inventor is that of a dental gel consisting of:
- from 83% to 89% by weight of hydrogen peroxide at various concentrations which can range between 1% and 3% by weight for an attacking therapy, down to 0.1% for a maintenance therapy;
- from 8% to 12% by weight of Methocel (carboxymethylcellulose);
- from 3% to 5% by weight of Carbopol (carboxypolymethylene);
- about 0.03% by weight of orthophosphoric acid; and optional colouring and/or flavouring additives.

Among the various embodiments, the one currently preferred by the inventor involves a dental gel in which:
- the hydrogen peroxide represents about 84% by weight from the concentrations referred to in the above preamble;
- the Carbopol is present in a proportion of about 4% by weight;
- the Methocel is present in a proportion of about 12% by weight;
- substances with known stabilizing action are present.

The abovementioned formulation and its variants mentioned were thoroughly investigated and were shown to have the following characteristics, which offer clear advantages over mixtures in paste form and likewise over liquid hydrogen peroxide, these being advantages which the inventor wishes to point out:
- By means of direct gelation it is possible to modify the concentration of the product, offering the orthodontist the possibility of accurately modifying the desired action, both as regards the amount and the concentration, by using a high concentration (3%) as an attacking therapy under the control of the orthodontist and a low concentration (0.1%) as a maintenance therapy for daily use.
- This product can be provided either in large-volume containers, or in single-dose or multi-dose bottles, these bottles allowing, inter alia, besides the practicality of use, a differentiated treatment, thereby also ensuring better stability of the product.
- This gel formulation makes it possible to accurately measure out the amount of product to be used which is capable of releasing a sufficient amount of oxygen which can carry out the mentioned anti-plaque activity.
- To be specific, about 3 ml of gel at the minimum concentration (0.1%) release just under 2 cm³ of oxygen, which is enough to be beneficial for the entire surface of the neck area of the teeth.
- This gel formulation used on a toothbrush allows enhancement of the anti-plaque effect already obtained by means of the mechanical action of the toothbrush, with a considerable advantage over the hydrogen peroxide solution in liquid form, both as regards the accuracy of the metering and as regards the practicality of use and the elimination of the risks of ingestion.
- The abovementioned mechanical action produces, with the heat generated from the friction, nascent oxygen which becomes fully available in situ.
- This gel formulation (direct gelation of hydrogen peroxide alone) gives an effect which is not "perturbed" by other substances, including saliva.
- This gel formulation "sticks to the teeth", giving rise to the abovementioned anti-plaque action directly "in situ" without the risk of becoming dispersed and of being accidentally ingested.
- Given the degree of viscosity of this formulation, it can moreover be used, where appropriate, directly in gingival pockets and remaining therein, using means of the type known for similar applications, such as, for example, syringes with a blunted needle.

In summary, the present invention enhances the abovementioned characteristics by offering stabilization of the product over time and immediate, localized release of oxygen.

Although the abovementioned gelling substances were found, in the course of the investigation, to be the most suitable for obtaining a correct level of oxidation of the dental gel and appropriate staying power of this gel over time, other similar substances or other compositions with different percentages by weight can be used to obtain the gelation of hydrogen peroxide, thereby obtaining a dental gel according to the invention which has equilibrated disinfecting action.

Lastly, the colouring and/or flavouring additives mentioned earlier can be chosen by specialists for the type of application or for the tastes of users of the dental gels according to the invention.

It goes without saying that all of the various embodiments thus obtained, if they refer to what is stated in the attached Claim 1, are included in the scope of the protection conferred by the present patent application.

It should be added that the process required to obtain the dental paste according to the invention is not far-removed from processes of the known type for obtaining the gelation of a component initially in liquid form.

## Claims

1. Dental gel for the hygiene and disinfection of the teeth and the gums, characterized in that it contains hydrogen peroxide obtained by direct gelation using gelling substances.

2. Dental gel according to Claim 1, characterized in that it contains:
• from 83% to 89% by weight of hydrogen peroxide solution;
• from 3% to 5% by weight of Carbopol (carboxypolymethylene);
• from 8% to 12% by weight of Methocel (carboxymethylcellulose);
• about 0.03% by weight of orthophosphoric acid;
• about 0.02% of methyl or propyl para-benzoate;
• optional colouring and/or flavouring additives.

3. Dental gel according to Claim 2, in which:
• the hydrogen peroxide, diluted to the desired concentration, represents 84% by weight;
• the Carbopol is present in a proportion of about 4% by weight;
• the Methocel is present in a proportion of about 12% by weight.

4. Dental gel according to either of Claims 2 and 3, in which the hydrogen peroxide concentration in the said solution can range from 3% to 0.1% by weight.
